# EUROPÄISCHE PATENTSCHRIFT

(11) **EP 3 492 142 B1**
(45) Veröffentlichungstag und Bekanntmachung des Hinweises auf die Patenterteilung: **02.06.2021**
(21) Anmeldenummer: 18199362.7
(22) Anmeldetag: 09.10.2018
(51) Int. Cl.: A61N 1/372, H01Q 1/27, H01Q 1/14, H01Q 1/24, A61N 1/375, H01Q 1/42, H01Q 9/42, H01Q 7/00

(54) **IMPLANTIERBARES MEDIZINELEKTRONISCHES GERÄT UND SENDE-/EMPFANGSANTENNE FÜR EIN SOLCHES**
IMPLANTABLE MEDICAL-ELECTRICAL DEVICE AND TRANSMIT/RECEIVE ANTENNA FOR SAME
APPAREIL ÉLECTRONIQUE MÉDICAL IMPLANTABLE ET ANTENNE D'ÉMISSION/ DE RÉCEPTION POUR UN TEL APPAREIL

(30) Priorität: 30.11.2017 EP 17204670
(43) Veröffentlichungstag der Anmeldung: 05.06.2019
(73) Patentinhaber: BIOTRONIK SE & Co. KG, 12359 Berlin (DE)
(72) Erfinder: Hartmann-Bax, Kathy, 14947 Nuthe-Urstromtal (DE); Ruschel, Marina, 12679 Berlin (DE)
(74) Vertreter: Galander, Marcus

(56) Entgegenhaltungen:
- EP-A1- 3 028 740
- WO-A1-2016/051206
- US-A1- 2013 116 763
- US-A1- 2014 364 714
- US-B2- 7 317 946

## Beschreibung

Die Erfindung betrifft ein implantierbares medizinelektronisches Gerät, welches einen Gerätekörper und einen hierauf aufgesetzten Header aufweist, mit einer Telemetrie-Baugruppe zur Signalübertragung nach und/oder von außerhalb des Körpers eines Patienten im implantierten Zustand des Gerätes, insbesondere zur drahtlosen bidirektionalen Kommunikation, wobei der Telemetrie-Baugruppe eine größtenteils aus einem langgestreckten Leiter räumlich geformte, im Bereich des Headers angeordnete Sende-/Empfangsantenne zugeordnet ist. Sie betrifft des Weiteren eine entsprechende Sende-/Empfangsantenne.

### Hintergrund

Es ist seit langem Praxis, dass implantierbare medizinelektronische Geräte über kurzreichweitige drahtlose Nachrichtenverbindungen, die sogenannte Telemetrie, im Körper aufgenommene Messwerte oder Zustandswerte des Gerätes nach außerhalb des Körpers des Patienten übermitteln bzw. von außen Steuer- bzw. Programmiersignale empfangen. Wie für jede Funkverbindung, sind für diese Kommunikation Antennen erforderlich. Die im implantierbaren Gerät vorgesehene Antenne sitzt außerhalb des elektromagnetisch abschirmenden Gerätegehäuses, also bei Geräten mit einem sogenannten Header (Kopfteil) im Bereich dieses Headers.

Wie alle Komponenten implantierbarer medizinelektronischer Geräte, sind auch die Telemetrie-Antennen Gegenstand ständiger Weiterentwicklung. Beispiele moderner Antennen dieser Art sind etwa in der US 7,317,946 B2 oder der EP 2 643 050 B1 beschrieben. Beide Druckschriften zeigen die Ausführung solcher Antennen mit einer Spiral- bzw. Mäanderstruktur zur Vergrößerung der wirksamen Leiterlänge unter gleichzeitiger Wahrung der erforderlichen Kompaktheit. Die US 7,317,946 B2 beschreibt im Übrigen, wie eine Sende-/Empfangsantenne der gattungsgemäßen Art platzsparend im Header eines implantierbaren Gerätes untergebracht werden und zugleich eine vorteilhafte Antennencharakteristik realisiert werden kann.

Das Dokument US 2014/0364714 A1 offenbart verschiedene Ausführungsformen für Antennen von implantierbaren medizinischen Geräten. Einige Antennen haben eine spiralförmige Struktur.

Das Dokument US 7,317,946 B2 offenbart weitere Ausführungsformen für Antennen von implantierbaren Medizingeräten, wobei einige Antennen eine mäanderförmige Struktur aufweisen.

Weitere Implantate mit Antennen sind in den Dokumenten US 2013/0116763 A1, EP 3 028 740 A1 und WO 2016/051206 A1 offenbart.

### Zusammenfassung

Der Erfindung liegt die Aufgabe zugrunde, ein weiter verbessertes implantierbares medizinelektronisches Gerät und insbesondere eine Sende-/Empfangsantenne für die Telemetriefunktion eines solchen Geräts anzugeben, wobei insbesondere ein geringer Montageaufwand und entsprechend niedrige Montagekosten mit vorteilhaften elektromagnetischen Eigenschaften kombiniert sein sollen.

Diese Aufgabe wird durch ein Gerät mit den Merkmalen des Anspruchs 1 und durch eine Sende-/Empfangsantenne mit den Merkmalen des Anspruchs 10 gelöst. Zweckmäßige Fortbildungen des Erfindungsgedankens sind Gegenstand der abhängigen Ansprüche.

Nach einem Aspekt ist ein implantierbares medizinelektronisches Gerät offenbart, welches einen Gerätekörper und einen hierauf aufgesetzten Header aufweist. Das Gerät weist des Weiteren eine Telemetrie-Baugruppe zur Signalübertragung nach und/oder von außerhalb des Körpers eines Patienten im implantierten Zustand des Gerätes auf, insbesondere zur drahtlosen bidirektionalen Kommunikation. Der Telemetrie-Baugruppe ist eine größtenteils aus einem langgestreckten Leiter räumlich geformte, im Bereich des Headers angeordnete Sende-/Empfangsantenne zugeordnet, welche derart konfiguriert ist, dass sie einen Formschluss mit der Außenkontur von mindestens einem Teil des Headers hat und hierdurch im Header fixiert ist. Ein erster Abschnitt des Leiters ist federelastisch ausgeführt und bildet eine Klammer, wobei die Klammer eine Anschlussbuchse im Header umgreift.

Das Gerät kann ein implantierbarer Herzschrittmacher, ein implantierbarer Kardioverter-Defibrillator (ICD - implantable cardioverter-defibrillator) oder ein implantierbarer Sensor sein.

Nach einem weiteren Aspekt ist eine Sende-/Empfangsantenne einer Telemetrie-Baugruppe eines implantierbaren medizinelektronischen Gerätes bereitgestellt. Das Gerät weist einen Gerätekörper und einen hierauf aufgesetzten Header auf. Die Sende-/Empfangsantenne ist aus einem langgestreckten Leiter räumlich geformt und derart konfiguriert, dass sie einen Formschluss mit der Außenkontur von einem Teil des Headers bildet und hierdurch im Header fixierbar ist. Ein erster Abschnitt des Leiters ist federelastisch ausgeführt und bildet eine Klammer, sodass mit der Klammer eine Anschlussbuchse im Header umgreifbar ist.

Erfindungsgemäß ist die Sende-/Empfangsantenne derart konfiguriert, dass sie einen Formschluss mit der Außenkontur von mindestens einem Teil des Headers hat und hierdurch auf dem Header fixiert ist. Es versteht sich, dass es neben der hier gemeinten Fixierung noch eine Verbindung eines Antennenendes mit einem Anschlussstift im Header gibt, die etwa als Schweiß-, Löt-, Leitklebeverbindungen oder alternativ auch als Crimp-, Steck- oder Klemmverbindung ausgeführt sein kann, die jedoch primär der elektrischen Verbindung zwischen der Telemetrie-Baugruppe und der Antenne dient.

Die federelastische Klammer ermöglicht eine besonders einfache Montage durch "Aufklipsen" der Antenne auf den ansonsten vormontierten Innenaufbau des Headers, ohne Werkzeug und in einem einzigen Fixierungsschritt. Sie ermöglicht zudem eine zuverlässige Fixierung auch bei Vorliegen geringfügiger Bauteiltoleranzen und vermeidet Handhabungsfehler bei der Montage.

Ein zweiter Abschnitt des Leiters kann auf einer Oberseite eines Buchsenanschlussblocks angeordnet sein, wobei der Buchsenanschlussblock die Anschlussbuchse aufweist.

An einer Seite des Buchsenanschlussblocks kann wenigstens ein Anschlussleiter angeordnet sein, welcher mit der Anschlussbuchse eine elektrisch leitfähige Verbindung hat. Der wenigstens eine Anschlussleiter stellt eine elektrische Verbindung zwischen einem in die Anschlussbuchse eingeführten Stecker und elektronischen Komponenten im Gerätekörper her.

Die Oberseite des Buchsenanschlussblocks und die Seite des Buchsenanschlussblocks können einen im Wesentlichen rechten Winkel (im Bereich der Fertigungstoleranzen, z. B. 90° plus/minus 2° oder 90° plus/minus 1°) einschließen. Aufgrund der Anordnung eines großen Teils der Antenne auf der Oberseite des Buchsenanschlussblocks schneidet sich die Ebene der Antenne nicht mit der Ebene, in welcher der (oder die) Anschlussleiter angeordnet ist (sind). Hierdurch sind die Wechselwirkungen der Antenne und des wenigstens einen Anschlussleiters mit einem Magnetfeld optimiert und die MRI-Kompatibilität (MRI - magnetic resonance imaging, Magnetresonanztomographie) des Geräts gewährleistet.

Der erste Abschnitt des Leiters kann zu dem auf der Oberseite des Buchsenanschlussblocks angeordneten zweiten Abschnitt des Leiters abgewinkelt sein. Der erste Abschnitt und der zweite Abschnitt können einen im Wesentlichen rechten Winkel (im Bereich der Fertigungstoleranzen, z. B. 90° plus/minus 2° oder 90° plus/minus 1°) einschließen.

Es kann vorgesehen sein, dass die Antenne genau eine Klammer aufweist, mit welcher die Antenne am Buchsenanschlussblock befestigt ist. Das "Aufklipsen" der einen Klammer an der Anschlussbuchse zusammen mit der Anordnung eines größten Teils der Antenne (zweiter Abschnitt des Leiters) auf der Oberseite des Buchsenanschlussblocks ermöglicht eine ausreichende Befestigung.

In einer weiteren Ausführung ist der den wesentlichen Teil des Antennenaufbaus bildende langgestreckte Leiter bandförmig, und zwar speziell mit einem Verhältnis zwischen Breite und Höhe des bandförmigen Leiters 4:1 oder mehr, insbesondere mehr als 8:1. Mit dieser Ausführung lässt sich die Antenne in technologisch besonders einfacher Weise als Stanzbiegeteil aus einem geeigneten elektrisch leitenden Metallblech oder einer elektrisch leitenden Metallfolie herstellen und hat eine, auf den Materialeinsatz bezogen, große Abstrahlfläche.

Neben der Ausführung als Stanzbiegeteil sind auch mannigfache andere Ausführungen der erfindungsgemäßen Antenne möglich, etwa als 3D-gedrucktes Sinterteil oder Metal-Injection-Mould (MIM)-Teil oder auch als durch ein Erosionsverfahren aus einem geeigneten elektrisch leitenden Metallblech oder einer elektrisch leitenden Metallfolie gebildetem Teil.

Zur Erreichung einer elektromagnetisch vorteilhaften Leiter-Gesamtlänge bei zugleich einfacher Herstellbarkeit und Montage ist die Sende-/Empfangsantenne in einer weiteren vorteilhaften Ausführung im größten Teil ihrer Erstreckung mit mäanderförmigem Verlauf in einer Ebene auf der Oberseite und/oder an einer Seitenfläche des Headers angeordnet. In einer Ausgestaltung dieser Ausführung sind mindestens in einem Teilabschnitt des mäanderförmigen Verlaufs Versteifungsbrücken in den Mäander-Bögen vorgesehen.

Die Klammer kann als Ω-förmige Klammer gebildet sein. Diese Ausführung eignet sich besonders für zahlreiche Geräte mit praktisch bewährten Header-Konstruktionen, die in ihrem Aufbau weitgehend genormte Anschlussbuchsen für Elektroden- und/oder Sensorleitungen umfassen. Neben einer guten mechanischen Befestigung der Antenne auf dem Buchsenanschlussblock wirkt die Ω-förmige Klammer auch als Antenne mit. Die Abstrahlcharakteristik und die Empfangscharakteristik der Antenne sind aufgrund der Ωförmigen Klammer verbessert. Hierdurch wird auch die MRI-Kompatibilität des Geräts weiter verbessert. Für abweichend gestaltete Header kann die Antennenkonstruktion vorteilhaft durch entsprechend angepasste Formgebung eines funktional entsprechenden Klammerabschnittes modifiziert sein, oder es können elastische Haken o.ä. vorgesehen sein.

Mit der erfindungsgemäßen Antenne lassen sich, jedenfalls in Ausführungsformen der Erfindung, insbesondere einer oder mehrere der nachfolgend genannten Vorteile erreichen:
- Die Antenne ermöglicht eine automatisierte Herstellung des kompletten Headers und trägt somit zu Effizienzsteigerungen und Kostensenkungen bei der Geräteproduktion bei.
- Durch eine im Hinblick auf den Materialeinsatz und die mechanischen Eigenschaften optimierte Leiterlänge und Abstrahlfläche sind die Antenneneigenschaften optimiert.
- Durch den Formschluss und die optionale abschnittsweise Elastizität der Antenne bezüglich der Headerkomponenten-Kontur werden ungewollte und leistungsbeeinträchtigende Verformungen der Antenne bei der Montage weitgehend verhindert und somit aufwändige Nacharbeiten und Ausschuss weitgehend vermieden.

### Beschreibung von beispielhaften Ausführungsformen

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1: eine perspektivische Ansicht einer herkömmlichen Sende-/Empfangsantenne im Header eines Einkammer-Herzschrittmachers,
- Fig. 2: eine perspektivische Gesamtansicht eines Einkammer-Herzschrittmachers mit darin eingezeichneter Ausführungsform der Sende-/Empfangsantenne,
- Fig. 3: eine perspektivische Ausschnittsdarstellung des Header-Aufbaus mit der Sende-/Empfangsantenne des Schrittmachers nach Fig. 2, und
- Fig. 4A - 4D: skizzenartige perspektivische Darstellungen weiterer Ausführungsformen der Sende-/Empfangsantenne.

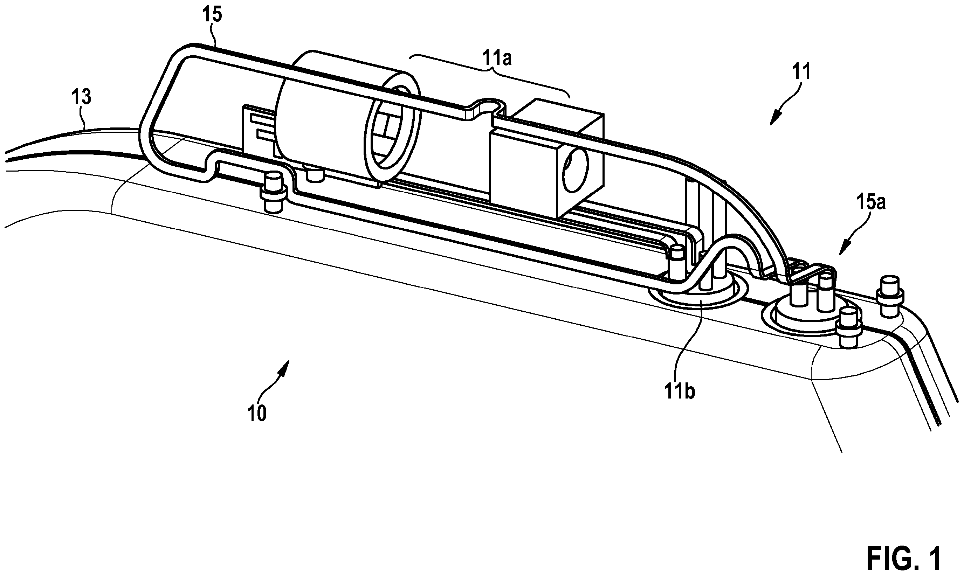

Fig. 1 zeigt in einer Ausschnittdarstellung einen Header 11 eines Herzschrittmachers 10 mit Anschlussbuchsen-Komponenten 11a und einem zugehörigen Anschlusselement 11b zur Verbindung mit (nicht dargestellten) elektronischen Komponenten in einem Gehäuse 13 des Schrittmachers. Weiterhin zeigt die Figur eine rahmenartige Sende-/Empfangsantenne 15 und ein dieser zugeordnetes Anschlusselement 15a. Es ist zu erkennen, dass die rahmenartige Sende-/Empfangsantenne 15 im Wesentlichen frei "schwebend" im Header 11 angeordnet und mechanisch nur am Anschlusselement 15a fixiert ist, welches natürlich außerdem ihrem elektrischen Anschluss dient.

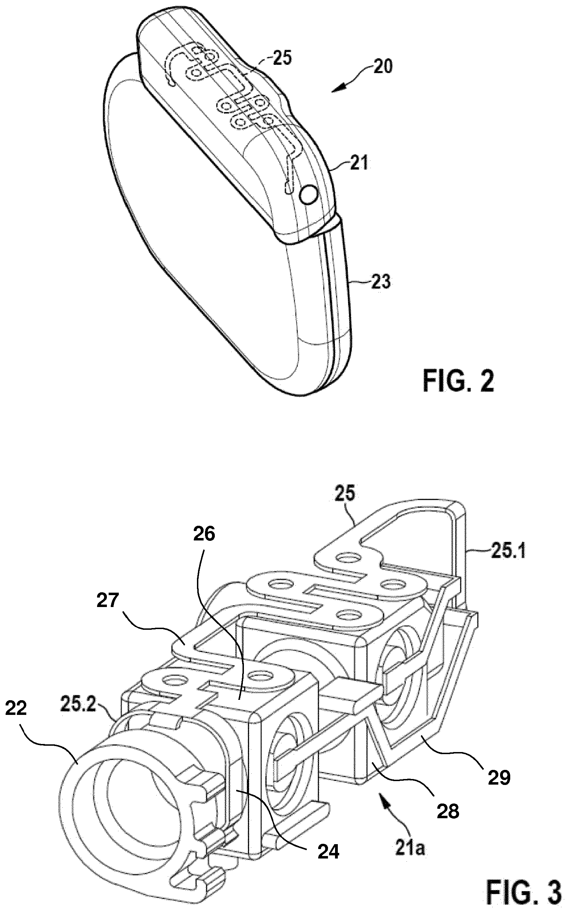

Fig. 2 zeigt eine Außenansicht eines erfindungsgemäß ausgestalteten Schrittmachers 20 mit einem Header 21 und einem Gehäuse 23, bei dem im Header 21 eine erfindungsgemäße Sende-/Empfangsantenne 25 angeordnet ist. Diese Sende-/Empfangsantenne 25 ist in Fig. 3 zusammen mit einem Buchsenanschlussblock 21a in perspektivischer Darstellung genauer gezeigt.

Ein erster Abschnitt 25.2 ist gegenüber einem zweiten Abschnitt 27 abgewinkelt. Der erste Abschnitt 25.2 und der zweite Abschnitt 27 bilden einen rechten Winkel. Es ist zu erkennen, dass die Antenne 25 über den größten Teil ihrer Erstreckung (zweiter Abschnitt 27) mit mäanderförmigem Verlauf in einer Ebene auf der Oberseite 26 des Buchsenanschlussblocks 21a liegt und lediglich ihre beiden Endabschnitte 25.1 und 25.2 außerhalb dieser Ebene liegen. Der Endabschnitt 25.1 führt zum (nicht dargestellten) Anschlusselement zur Verbindung mit einer zugehörigen Telemetrie-Baugruppe im Schrittmachergehäuse. Der erste Abschnitt 25.2 ist als Ω-förmige Klammer 24 gestaltet, die eine (z. B. zylinderförmige) Anschlussbuchse 22 des Buchsenanschlussblocks 21a elastisch federnd umgreift. Es ist auch zu erkennen, dass innerhalb der Mäander des Antennenverlaufes brückenartige Verbindungen vorgesehen sind, die eine Erhöhung der Biegesteifigkeit des Antennenaufbaus bewirken. Weiterhin ist zu erkennen, dass die Sende-/Empfangsantenne 25 als flaches Stanzbiege- oder Formteil ausgebildet ist. An einer Seite 28 des Buchsenanschlussblocks 21a ist ein Anschlussleiter 29 angeordnet, der eine elektrische Verbindung zu der Anschlussbuchse 22 aufweist. Die Oberseite 26 des Buchsenanschlussblocks 21a und die Seite 28 des Buchsenanschlussblocks 21a schließen einen rechten Winkel ein.

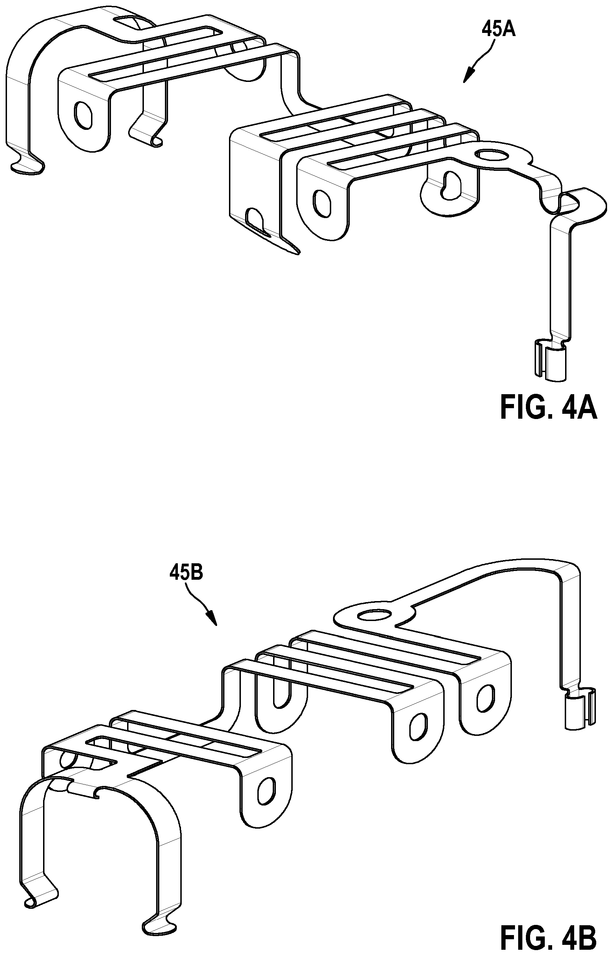

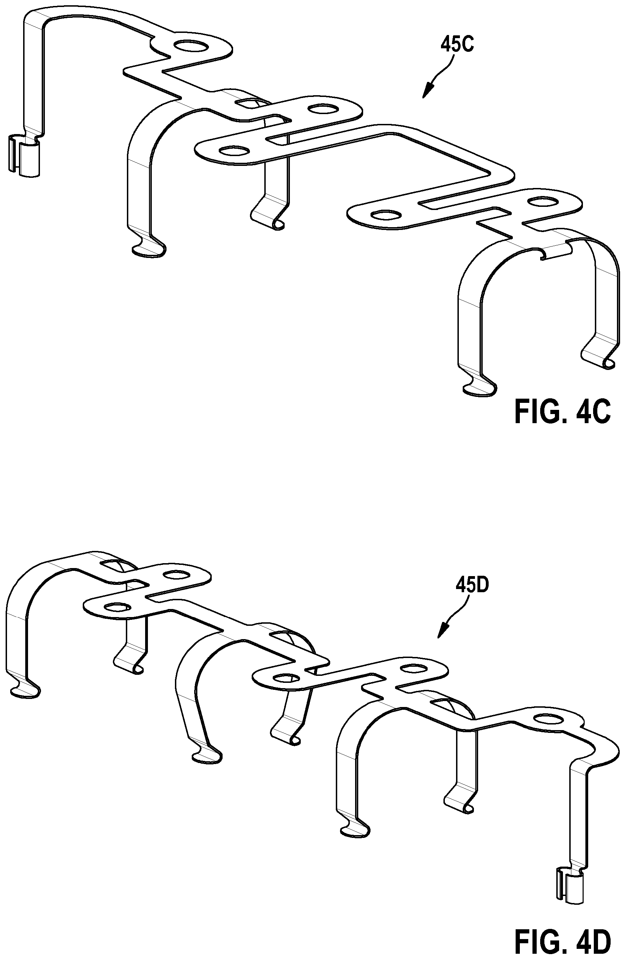

Die Figuren 4A bis 4D zeigen weitere Ausführungsformen von Sende-/Empfangsantennen 45A - 45D. Bei den Antennen 45A nach Fig. 4A und 45B nach Fig. 4B sind jeweils die Endabschnitte der Mäander des Antennen-Mittenabschnittes rechtwinklig abgekantet, womit ein zusätzlicher Formschluss mit einer im montierten Zustand darunter liegenden (nicht dargestellten) Buchsenbaugruppe des Headers gebildet werden kann. Die Sende-/Empfangsantennen 45C nach Fig. 4C und 45D nach Fig. 4D haben jeweils mehr als einen Klammerabschnitt und fixieren somit im montierten Zustand die jeweilige Antenne an mehreren Stellen durch elastisch federndes Umgreifen eines zylindrischen Abschnitts der jeweiligen, unter der Antenne liegenden (wiederum nicht dargestellten) Header-Baugruppe. Es ist darauf hinzuweisen, dass die Klammerabschnitte in allen Ausführungen neben ihrer mechanischen Fixierungsfunktion auch eine elektrische Funktion haben und die Antennencharakteristik vorteilhaft beeinflussen.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Implantierbares medizinelektronisches Gerät (20), welches einen Gerätekörper (23) und einen hierauf aufgesetzten Header (21) aufweist, mit einer Telemetrie-Baugruppe zur Signalübertragung nach und/oder von außerhalb des Körpers eines Patienten im implantierten Zustand des Gerätes, insbesondere zur drahtlosen bidirektionalen Kommunikation, wobei der Telemetrie-Baugruppe eine größtenteils aus einem langgestreckten Leiter räumlich geformte, im Bereich des Headers angeordnete Sende-/Empfangsantenne (25; 45A - 45D) zugeordnet ist, welche derart konfiguriert ist, dass sie einen Formschluss mit der Außenkontur von mindestens einem Teil (21a) des Headers hat und hierdurch im Header fixiert ist, wobei
- ein erster Abschnitt (25.2) des Leiters federelastisch ausgeführt ist und eine Klammer (24) bildet, wobei die Klammer (24) eine Anschlussbuchse (22) im Header (21) umgreift,
- ein zweiter Abschnitt (27) des Leiters auf einer Oberseite (26) eines Buchsenanschlussblocks (21a) angeordnet ist, wobei der Buchsenanschlussblock (21a) die Anschlussbuchse (22) aufweist, und
- an einer Seite (28) des Buchsenanschlussblocks (21a) wenigstens ein Anschlussleiter (29) angeordnet ist, welcher mit der Anschlussbuchse (22) eine elektrisch leitfähige Verbindung hat.

2. Gerät nach Anspruch 1, wobei die Oberseite (26) des Buchsenanschlussblocks (21a) und die Seite (28) des Buchsenanschlussblocks (21a) einen im Wesentlichen rechten Winkel einschließen.

3. Gerät nach Anspruch 1 oder 2, wobei der erste Abschnitt (25.2) des Leiters zu dem auf der Oberseite (26) des Buchsenanschlussblocks (21a) angeordneten zweiten Abschnitt (27) des Leiters abgewinkelt ist.

4. Gerät nach einem der vorangehenden Ansprüche, wobei der langgestreckte Leiter bandförmig ist.

5. Gerät nach Anspruch 4, wobei das Verhältnis zwischen Breite und Höhe des bandförmigen Leiters 4:1 oder mehr, insbesondere mehr als 8:1, beträgt.

6. Gerät nach einem der vorangehenden Ansprüche, wobei die Sende-/Empfangsantenne (25; 45A - 45D) als Stanzbiegeteil, 3D-gedrucktes Sinterteil oder Metal-Injection-Mould-Teil ausgeführt ist.

7. Gerät nach einem der vorherigen Ansprüche, wobei die Sende-/Empfangsantenne im zweiten Abschnitt (27) des Leiters und damit im größten Teil ihrer Erstreckung mit einem mäanderförmigem Verlauf in einer Ebene auf der Oberseite (26) des Buchsenanschlussblocks (21a) angeordnet ist.

8. Gerät nach Anspruch 7, wobei mindestens in einem Teilabschnitt des mäanderförmigen Verlaufs Versteifungsbrücken in den Mäander-Bögen vorgesehen sind.

9. Gerät nach einem der vorangehenden Ansprüche, wobei die Klammer (24) als Ωförmige Klammer gebildet ist.

10. Sende-/Empfangsantenne (25; 45A - 45D) einer Telemetrie-Baugruppe eines implantierbaren medizinelektronischen Gerätes (20), welches einen Gerätekörper (23) und einen hierauf aufgesetzten Header (21) aufweist, wobei die Sende-/Empfangsantenne aus einem langgestreckten Leiter räumlich geformt und derart konfiguriert ist, dass sie einen Formschluss mit der Außenkontur von mindestens einem Teil (21a) des Headers (21) bildet und hierdurch im Header (21) fixierbar ist, wobei ein erster Abschnitt (25.2) des Leiters federelastisch ausgeführt ist und eine Klammer (24) bildet, sodass mit der Klammer (24) eine Anschlussbuchse (22) im Header (21) umgreifbar ist, wobei die Klammer (24) als Ω-förmige Klammer gebildet ist.

11. Sende-/Empfangsantenne nach Anspruch 10, wobei der erste Abschnitt (25.2) des Leiters zu einem zweiten Abschnitt (27) des Leiters abgewinkelt ist.

## Claims

1. An implantable electronic medical device (20), comprising a device body (23) and a header (21) placed thereon, including a telemetry assembly for signal transmission to and/or from outside the body of a patient when the device is implanted, in particular for wireless bidirectional communication, wherein a transmit/receive antenna (25; 45A to 45D) is assigned to the telemetry assembly, wherein the transmit/receive antenna (25; 45A to 45D) is essentially physically formed of an elongated conductor and is arranged in a region of the header, wherein the transmit/receive antenna (25; 45A to 45D) is configured to have a form fit with an outer contour of at least a portion (21a) of the header and is fixed thereby in the header, wherein
- a first section (25.2) of the conductor has a spring elastic design and forms a bracket (24), and wherein the bracket (24) embraces a connector (22) in the header (21),
- a second section (27) of the conductor is arranged on an upper face (26) of a connection block (21a), the connection block (21a) comprising the connector (22), and,
- at least one connecting conductor (29), which has an electrically conductive connection with the connector (22), is arranged on a side (28) of the connection block (21a).

2. The device according to claim 1, wherein the upper face (26) of the connection block (21a) and the side (28) of the connection block (21a) include a substantially right angle.

3. The device according to claim 1 or 2, wherein the first section (25.2) of the conductor is angled with respect to the second section (27) of the conductor, which is arranged on the upper face (26) of the connection block (21a).

4. The device according to any one of the preceding claims, wherein the elongated conductor is ribbon-shaped.

5. The device according to claim 4, wherein the ratio of the width to the height of the ribbon-shaped conductor is 4:1 or more, and in particular more than 8:1.

6. The device according to any one of the preceding claims, wherein the transmit/receive antenna (25; 45A to 45D) is designed as a bent stamping, a 3D printed sintered part or a metal injection molded part.

7. The device according to any one of preceding claims, wherein the transmit/receive antenna (25; 45A to 45D) is arranged in the second section (27) of the conductor, and wherein the transmit/receive antenna (25; 45A to 45D) comprises a meander-shaped progression which is arranged in a plane on the upper face (26) of the connection block (21a) in the majority of the extension of the transmit/receive antenna (25; 45A to 45D).

8. The device according to claim 7, wherein reinforcement bridges are provided in the meander bends at least in a sub-section of the meander-shaped progression.

9. The device according to any one of the preceding claims, wherein the bracket (24) is designed as a Ω-shaped bracket.

10. A transmit/receive antenna (25; 45A to 45D) of a telemetry assembly of an implantable electronic medical device (20), which comprises a device body (23) and a header (21) placed thereon, wherein the transmit/receive antenna is physically formed of an elongated conductor and configured to form a form fit with the outer contour of at least a portion (21a) of the header (21), and is fixable thereby in the header (21), a first section (25.2) of the conductor having a spring elastic design and forming a bracket (24), so that a connector (22) in the header (21) is embracable by the bracket (24), wherein the bracket (24) is designed as a Ω-shaped bracket..

11. The transmit/receive antenna according to claim 10, wherein the first section (25.2) of the conductor is angled with respect to a second section (27) of the conductor.

## Revendications

1. Appareil médical électronique implantable (20), lequel présente un corps d'appareil (23) et un bloc d'en-tête (21) posé dessus, avec un composant de télémétrie pour la transmission de signaux vers, et/ou en provenance de l'extérieur du corps d'un patient à l'état implanté de l'appareil, notamment pour la communication bidirectionnelle sans fil, où une antenne d'émission et de réception (25 ; 45A à 45D), formée dans l'espace en plus grande partie à base d'un conducteur allongé, est associée au composant de télémétrie, disposée dans la région du bloc d'en-tête, laquelle est conçue de telle manière qu'elle a une complémentarité de forme avec le contour extérieur d'au moins une partie (21a) du bloc d'en-tête et se trouve ainsi fixée dans le bloc d'en-tête, où
- un premier segment (25.2) du conducteur est conçu élastique comme un ressort et forme une pince (24), où la pince (24) entoure une fiche de connexion (22) dans le bloc d'en-tête (21),
- un deuxième segment (27) du conducteur est disposé sur une face supérieure (26) d'un bloc de fiche de connexion (21a), où le bloc de fiche de connexion (21a) présente la fiche de connexion (22), et
- au moins un conducteur de connexion (29) est disposé sur un côté (28) du bloc de fiche de connexion (21a), lequel possède une liaison électriquement conductrice avec la fiche de connexion (22).

2. Appareil selon la revendication 1, dans lequel la face supérieure (26) du bloc de fiche de connexion (21a) et le côté (28) du bloc de fiche de connexion (21a) forment essentiellement un angle droit.

3. Appareil selon la revendication 1 ou la revendication 2, dans lequel le premier segment (25.2) du conducteur forme un angle avec le deuxième segment (27) du conducteur disposé sur la face supérieure (26) du bloc de fiche de connexion (21a).

4. Appareil selon l'une des revendications précédentes, dans lequel le conducteur allongé est en forme de bande.

5. Appareil selon la revendication 4, dans lequel le rapport entre la largeur et la hauteur du conducteur en forme de bande est égal ou supérieur à 4 : 1, notamment supérieur à 8 : 1.

6. Appareil selon l'une des revendications précédentes, dans lequel l'antenne d'émission et de réception (25 ; 45A à 45D) est conçue sous forme d'une pièce emboutie et pliée, d'une pièce frittée à impression 3D ou d'une pièce en métal moulée par injection.

7. Appareil selon l'une des revendications précédentes, dans lequel l'antenne d'émission et de réception est disposée dans le deuxième segment (27) du conducteur et par conséquent dans la plus grande partie de son extension avec une évolution en forme de méandres dans un plan sur la face supérieure (26) du bloc de fiche de connexion (21a).

8. Appareil selon la revendication 7, dans lequel des pontages de rigidification sont prévus dans les boucles des méandres au moins dans un segment partiel de l'évolution en forme de méandres.

9. Appareil selon l'une des revendications précédentes, dans lequel la pince (24) est conçue comme une pince en forme de Ω.

10. Antenne d'émission et de réception (25 ; 45A à 45D) d'un composant de télémétrie d'un appareil électronique médical implantable (20), lequel présente un corps d'appareil (23) et un bloc d'en-tête (21) posé dessus, où l'antenne d'émission et de réception est formée dans l'espace à base d'un conducteur allongé et est conçue de telle manière qu'elle a une complémentarité de forme avec le contour extérieur d'au moins une partie (21a) du bloc d'en-tête (21) et peut être ainsi fixée dans le bloc d'en-tête (21), où un premier segment (25.2) du conducteur est conçu élastique comme un ressort et forme une pince (24), de sorte qu'une fiche de connexion (22) peut être entourée en prise dans la pince (24), dans le bloc d'en-tête (21), où la pince (24) est conçue comme une pince en forme de Ω.

11. Antenne d'émission et de réception selon la revendication 10, dans laquelle le premier segment (25.2) du conducteur forme un angle avec un deuxième segment (27) du conducteur.
